# EUROPÄISCHE PATENTSCHRIFT

(11) **EP 3 216 492 B1**
(45) Veröffentlichungstag und Bekanntmachung des Hinweises auf die Patenterteilung: **12.05.2021**
(21) Anmeldenummer: 16159354.6
(22) Anmeldetag: 09.03.2016
(51) Int. Cl.: A61N 5/06, G02B 6/02

(54) **FASERMIKROELEKTRODE**
FIBRE MICRO-ELECTRODE
MICROELECTRODE EN FIBRES

(43) Veröffentlichungstag der Anmeldung: 13.09.2017
(73) Patentinhaber: Technische Hochschule Mittelhessen, 35390 Giessen (DE)
(72) Erfinder: Schanze, Thomas, 35037 Marburg (DE)
(74) Vertreter: Stumpf, Peter

(56) Entgegenhaltungen:
- KR-A- 20150 102 809
- US-A1- 2016 059 030
- US-B1- 8 632 577
- SANYUAN CHEN ET AL: "Paper;A fiber-based implantable multi-optrode array with contiguous optical and electrical sites;A fiber-based implantable multi-optrode array with contiguous optical and electrical sites", JOURNAL OF NEURAL ENGINEERING, INSTITUTE OF PHYSICS PUBLISHING, BRISTOL, GB, Bd. 10, Nr. 4, 24. Juli 2013 (2013-07-24), Seite 46020, XP020248757, ISSN: 1741-2552, DOI: 10.1088/1741-2560/10/4/046020
- LYUBOV V. DORONINA-AMITONOVA ET AL: "Fiber-optic probes for in vivo depth-resolved neuron-activity mapping", JOURNAL OF BIOPHOTONICS, Bd. 3, Nr. 10-11, 2. Oktober 2010 (2010-10-02), Seiten 660-669, XP055299603, DE ISSN: 1864-063X, DOI: 10.1002/jbio.201000041
- JIAYI ZHANG ET AL: "Integrated device for optical stimulation and spatiotemporal electrical recording of neural activity in light-sensitized brain tissue; Integrated device for optical stimulation and spatiotemporal electrical recording of neural activity", JOURNAL OF NEURAL ENGINEERING, INSTITUTE OF PHYSICS PUBLISHING, BRISTOL, GB, Bd. 6, Nr. 5, 1. September 2009 (2009-09-01), Seite 55007, XP020165975, ISSN: 1741-2552, DOI: 10.1088/1741-2560/6/5/055007
- Giancarlo Chesini ET AL: "All-fiber devices based on photonic crystal fibers with integrated electrodes", Optics Express, vol. 17, no. 3, 2 February 2009 (2009-02-02), page 1660, XP055299222, ISSN: 2161-2072, DOI: 10.1364/OE.17.001660

## Beschreibung

Die vorliegende Erfindung betrifft eine neuartige Fasermikroelektrode, welche zum Beispiel zur optischen Stimulation und gleichzeitiger elektrischer Messung von neuronalen Interaktionen z.B. im Gehirn nutzbar ist.

### Stand der Technik

Fasermikroelektroden bestehen aus elektrischen Leitern (Drähten), die in einen Isolator, vorzugsweise eine Glasfaser, eingebettet sind. Das Glas (üblicherweise Quarzglas) dient als Trägermaterial und Isolator für die Leiter, die z.B. aus einer Platin-Wolfram-Legierung bestehen. An der Faserspitze stellen die Leiter elektrischen Kontakt zum Zielorgan her, am Faserende werden sie mit elektronischen Systemen zur Signalableitung oder zur elektrischen Stimulation verbunden. So besteht eine Fasermikroelektrode aus vier elektrischen Leitern bzw. Kontakten. Bei der Herstellung werden Glas- und Metallstäbe, deren Durchmesser im Millimeterbereich liegen, lokal zu einer Schmelze erhitzt, die dann zu einer dünnen Faser ausgezogen wird.

Der Herstellungsprozess einer Fasermikroelektrode ist technisch aufwändig, da verschiedene Materialparameter, z.B. Wärmeausdehnungskoeffizienten, berücksichtigt werden müssen. Die Vorteile von (Quarz)glas-Fasermikroelektroden sind gute Handhabbarkeit, große Robustheit und Biokompatibilität, die für medizinische und neurowissenschaftliche Applikationen relevant ist. Insbesondere sind sie auch erfolgreich klinisch erprobt worden.

Für die Übertragung von Informationen sind als Lichtleiter funktionierende Glasfasern seit langem bekannt und in der Verwendung. Eine Glasfaser ist eine aus Glas bestehende lange dünne Faser. In Faserrichtung kann sich Licht nahezu ungehindert ausbreiten. Bei einfachen Lichtleiter-Fasern genügt der Übergang Glas-Luft, um das Licht in der Faser mittels Totalreflexion zu führen. Durch einen radial nach außen abnehmenden Brechungsindex, der stetig oder stufig sein kann, können die Lichtführungseigenschaften gezielt eingestellt werden. Diese Lichtleitereigenschaft wird in vielen Anwendungen eingesetzt. Bei der Herstellung werden Glasfasern aus Glasschmelzen gezogen.

Fasermikroelektroden im Allgemeinen sind seit einigen Jahren im Einsatz und werden z.B. zur Messung der neuronalen Interaktion im Gehirn verwendet.

Übliche Fasermikroelektroden eignen sich nicht als Lichtleiter. Gründe sind die Störungen der Ausbreitung des Lichts durch die in die Faser eingebetteten Drähte. Eine gezielte und hinreichend hohe Manipulation des Brechungsindex um den Lichtleiterkern durch die Verwendung geeignet dotierter Quarzstäbe ist aufgrund der dadurch entstehenden ungünstigen thermo-mechanischen Parameterkonstellationen bisher nicht realisiert. Zum anderen sind die erzielbaren Brechzahlgradienten häufig unzureichend, so dass schon geringfügige Faserkrümmungen zu nicht tolerierbaren Lichtverlusten führen.

Die Schrift SANYUAN CHEN ET AL: "A fiber-based implantable multi-optrode array with contiguous optical and electrical sites", (DOI: 10.1088/1741-2560/10/4/046020) offenbart eine Fasermikroelektrode mit einen lichtleitenden Kern und einen Glasmantel um den lichtleitenden Kern mit wenigstens einem elektrischen Leiter. Wobei sich dieser zwischen der Glasfaser und dem Glasmantel befindet. Dieser Aufbau führt zu einer starken Wechselwirkung von innerhalb des lichtleitenden Kern geführtem Licht mit dem in einem elektrischen Leiter geführten elektrischen Signalen.

Die Schrift LYUBOV V. DORONINA-AMITONOVA ET AL: "Fiber-optic probes for in vivo depth-resolved neuron-actitrity mapping", (DOI: 10.1002/jbio.201000041) offenbart Glasfasern, die zur Verbesserung der optischen Eigenschaften von einem photonischen Kristall umgeben sind.

Die Druckschrift KR 2015 0102809 A offenbart eine Fasermikroelektrode gemäß dem Oberbegriff von Anspruch 1.

### Aufgabe

Aufgabe der vorliegenden Erfindung ist es, eine Fasermikroelektrode bereitzustellen, welche neben elektrischen auch optische Signale gleichzeitig, zuverlässig, mit geringer Dämpfung und mit geringer Wechselwirkung von innerhalb der Glasfaser geführtem Licht mit dem in einem elektrischen Leiter geführten elektrischen Signalen leiten kann.

### Lösung der Aufgabe

Gelöst wird diese Aufgabe durch eine Vorrichtung mit den Merkmalen des Anspruchs 1. Vorteilhafte Ausführungsformen und Weiterbildungen der vorliegenden Erfindung ergeben sich aus den Unteransprüchen.

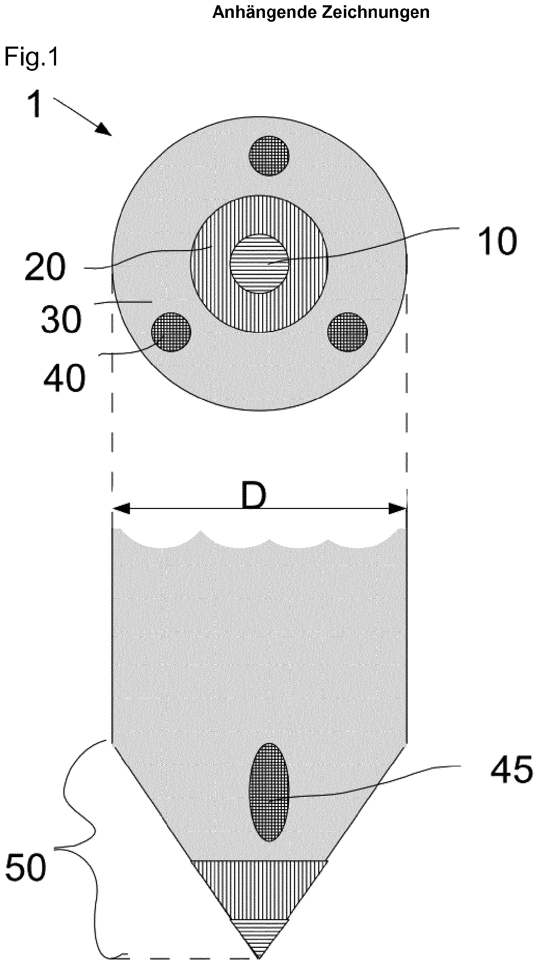

Die erfindungsgemäße Fasermikroelektrode 1 umfasst wenigstens einen lichtleitenden Kern 10, einen Glasmantel 30 um den lichtleitenden Kern 10. Der Glasmantel umfasst dabei wenigstens einen elektrischen Leiter 40. Wenigstens ein lichtleitender Kern 10 ist hierbei von einen photonischen Kristall 20 umgeben, wobei sich der phonische Kristall 20 zumindest abschnittsweise innerhalb des Glasmantels 30 befindet. Dabei ist der wenigstens eine elektrischen Leiter 40 entlang der Fasermikroelektrode 1 angeordnet ist und befindet sich innerhalb des Glasmantels 30. Eine schematische Darstellung einer bevorzugten Ausführungsform der erfindungsgemäßen Fasermikroelektrode 1 zeigt die Abbildung Fig.1.

Der Durchmesser D der Fasermikroelektrode 1 beträgt typischerweise etwa 100 µm bevorzugt 15 µm bis 300 µm, besonders bevorzugt 20 µm bis 200 µm. Die maximale Länge kann bei der Herstellung einige 100 m (bis zu 500 m) erreichen.

In einer alternativen Ausführungsform umfasst die Fasermikroelektrode 1 eine Beschichtung 90. Diese umhüllt den Glasmantel 30 zumindest abschnittsweise.

Die Beschichtung 90 kann hierbei vorzugsweise Acrylat, Silikon, Polyimid und/oder Parylene umfassen. Sie hat die Aufgabe die Eigenschaften der Fasermikroelektrode 1 positiv zu beeinflussen. So verringert die Beschichtung 90 die Reibung und/oder stellt eine besonders wasserabweisende Oberfläche da. Das erhöht die Bioinertheit bzw. Biostabilität der Fasermikroelektrode 1, sodass es zu einer geringeren chemischen und/oder biologischen Wechselwirkung mit biologischen Material kommt.

Der lichtleitende Kern 10 dient der Führung von Licht durch die Fasermikroelektrode 1. Er besteht aus einem optisch transparenten Material. Dies kann ein Glas, ein Polymer, Luft (bzw. andere transparente Gasgemische) und/oder eine Kombination dieser Materialien umfassen. Der lichtleitende Kern 10 kann in einer alternativen Ausführungsform auch aus einem photonischen Kristall aufgebaut sein. Über die Spitze 50 der Fasermikroelektrode kann Licht in den lichtleitenden Kern 10 ein-oder ausgekoppelt werden. Dies geschieht wie bei üblichen Glasfasern. Das verwendete Licht ist in der Regel sichtbares Licht zwischen 380 nm bis 780 nm Wellenlänge oder Infrarot zwischen 780 nm und 5 µm Wellenlänge. Die Strahlung ist üblicherweise monochromatisch und stammt beispielsweise aus einem Laser oder einer Leuchtdiode.

Der Glasmantel 30 umgibt den lichtleitende Kern 10 und den photonischen Kristall 20. Er besteht aus einem Glas, üblicherweise einem Quarzglas. Er dient als mechanischer Schutz des lichtleitenden Kerns. Vorzugsweise dient er zusätzlich auch als mechanischer Schutz und als elektrische Isolierung des elektrischen Leiters 40, welcher sich innerhalb des Glasmantels 30 befindet.

Der photonische Kristall 20 umgibt den lichtleitenden Kern 10 der Fasermikroelektrode 1. Er hat die Aufgabe, die Ausbreitung des Lichts in Faserrichtung so zu beeinflussen, dass keine Wechselwirkung des durch den lichtleitenden Kern 10 geführten Lichts mit dem elektrischen Leiters 40 auftritt. Weiterhin dient der photonische Kristall dazu die Dämpfung des geführten Lichts innerhalb der Fasermikroelektrode 1 gering zu halten. Das ermöglicht eine stabile, gleichzeitige Signalübertragung optischer und elektrischer Signale innerhalb der Fasermikroelektrode 1.

Photonische Kristalle sind natürlich in transparenten Festkörpern vorkommende oder künstlich erzeugte periodische und/oder nicht periodische Strukturen des Brechungsindex, die u. a. durch Beugung und Interferenz die Bewegung von Photonen (in der Regel sichtbares Licht zwischen 380 nm bis 780 nm Wellenlänge oder Infrarot zwischen 780 nm und 5 µm Wellenlänge) beeinflussen. Dadurch wird es nicht nur möglich, Licht auf Abmessungen, welche in der Größenordnung der Wellenlänge liegen, zu führen, sondern auch zu filtern und wellenlängenselektiv zu reflektieren. Photonische Kristalle sind nicht zwingend kristallin - die Bezeichnung stammt von analogen Beugungs- und Reflexionseffekten von Röntgenstrahlung in Kristallen aufgrund deren Gitterkonstanten.

Die Strukturabmessungen von photonischen Kristallen sind gleich oder größer eines Viertels der zugehörigen Wellenlänge der Photonen, sie liegen also im Bereich von Bruchteilen eines Mikrometers bis mehrere Mikrometer. Photonische Kristalle lassen sich von Interferenzschichten und Beugungsgittern dadurch abgrenzen, dass sie drei- oder auch eindimensional sein können und dass sie steuerbar sein können. Es handelt sich um periodische dielektrische Strukturen, deren Periodenlänge so eingestellt ist, dass sie die Ausbreitung von elektromagnetischen Wellen in ähnlicher Weise beeinflussen wie das periodische Potential in Halbleiterkristallen die Ausbreitung von Elektronen. Sie zeigen daher einzigartige optische Eigenschaften, wie beispielsweise Bragg-Reflexion von sichtbarem Licht. Insbesondere entsteht analog zur Ausbildung der elektronischen Bandstruktur eine photonische Bandstruktur, die Bereiche verbotener Energie aufweisen kann, in denen sich elektromagnetische Wellen nicht innerhalb des Kristalls ausbreiten können (photonische Bandlücken, PBG = englisch: photonic band gap). Photonische Kristalle können also in gewisser Weise als das optische Analogon zu elektronischen Halbleitern, also als "optische Halbleiter" angesehen werden.

Der photonische Kristall 20 besteht aus einem Halbleiter, Glas, (transparenter) Keramik und/oder Polymer. Typische Halbleitermaterialien sind insbesondere Ge, Si, **α**-Sn, C (Fullerene), B, Se, Te, GaP, GaAs, InP, InSb, InAs, GaSb, GaN, AIN, InN, AlxGa1-xAs, InxGa1-xN, Tetracen, Pentacen, Phthalocyanine, Polythiophene, PTCDA, MePTCDI, Chinacridon, Acridon, Indanthron, Flavanthron, Perinon und Alq3 (tris-8-hydroxy-quinolato aluminum).

Typische in der Optik eingesetzte Gläser sind: Quarzglas, Fluor-Kronglas, Kronglas, Borosilikat-Kronglas, Barium-Kronglas, Leichtflintglas, Schwerkronglas und Flintglas. Optisch-transparente Keramikmaterialen sind beispielsweise Perlucor, Al₂O₃, YAG, Nd:YAG und Nd:YVO4 erwähnt.

Typtische optische Polymere sind: Polymethylmethacrylat (PMMA), Polyetherketon, Polymethylmethacrylat, Polyphenylenvinyl, Polycarbonat, Polyacetylen und Polytetrafluorethylen.

Der photonische Kristall 20 ist vorzugsweise kristallin ausgebildet. Der Durchmesser d des photonischen Kristalls 20 liegt, je nach Ausführung, bei 10-30 µm.

In einer besonderen Ausführungsform ist wenigstens ein photonischer Kristall 20 als ein zweidimensionaler Kristall ausgebildet.

In einer weiteren Ausführungsform ist der photonische Kristall 20 aus photonischen Kristall-Fasern (PCF, *photonic-crystal fiber)* ausgeführt.

In einer weiteren Ausführungsform umfasst die Fasermikroelektrode 1 eine Trennschicht 80, welche den photonischen Kristall 20 zumindest abschnittsweise umhüllt. Diese Trennschicht 80, welche aus Quarzglasstäbe oder -röhrchen mit anderen optischen Eigenschaften (Brechzahl, Farbe) als der photonische Kristall 20 besteht, dient dazu das optische oder elektrische Übersprechen gezielt zu verringern. Die Trennschicht kann zusätzlich oder alternativ zur Beschichtung 90 verwendet werden.

Der elektrische Leiter 40 innerhalb der Fasermikroelektrode 1 dient zur Leitung elektrischen Signalen. Der elektrische Leiter 40 ist so ausgebildet, dass er über elektrische Kontakte 45 an der Oberfläche der Fasermikroelektrode 1 oder über die Spitze 50 der Fasermikroelektrode elektrische Signale empfangen oder senden kann. Die Fasermikroelektrode 1 kann auch mehrere elektrische Leiter umfassen. Ublich sind 2 bis 4 elektrische Leiter. Es ist aber auch eine größere Anzahl elektrischer Leiter möglich.

Der elektrischen Leiters 40, besteht z.B. aus einer Platin-Wolfram-Legierung (Pt/W 950/50). Andere geeignete Materialien sind beispielsweise Iridium Kupfer, Gold, Platin, Eisen oder Legierungen dieser Metalle. Der Durchmesser liegt bevorzugt bei 10 bis 20 µm.

Zur Fertigung der erfindungsgemäßen Fasermikroelektrode werden Glasstäbe, Glasrohre und Metallstabe, die vorzugsweise biokompatibel sind, geeignet zusammengefügt, erhitzt und ausgezogen Dies geschieht wie bei der Herstellung von herkömmlichen Fasermikroelektroden. Der photonische Kristall wird beispielsweise durch eine gezielte Anordnung von Rohren und/oder Stäben aus Halbleiter, Quarzglas, Keramik und/oder einem Polymer realisiert. Hierbei können auch unterschiedliche Gläser Verwendung finden. Weiterhin können die Glasrohre und Glasstäbe dotiert, z.B. mit Germanium, oder beschichtet sein. Die thermischen Eigenschaften aller Materialien sind korrespondierend zu wählen, z.B. nahezu identische Schmelzpunkte. Dies erleichtert die Herstellung.

Der Übergang vom Glaszustand der Quarz-Faser in den flüssigen Zustand ist nicht dasselbe wie Schmelzen. Bei elektrisch geschmolzenem Quarzglas liegt die Glasübergangstemperatur *T_{G}* bei 1710 °C. Der Schmelzpunkt von reinem Platin liegt bei 1772 °C. Für eine Platin-Wolfram-Legierung mit 95 % Pt und 5 % W (Pt/W 950/50 92/W8) ist der Schmelzbereich 1840-1860 °C. Der Rohling der Fasermikroelektrode der beispielsweise aus Quarzröhren, Quarzstäben und Pt/W-Drähten besteht wird für die Herstellung der Fasermikroelektrode auf die Schmelztemperatur der Drähte gebracht und sodann zu einer dünnen Faser ausgezogen. Dies ist möglich, da bei dieser Temperatur der Quarz zwar weich, aber noch nicht flüssig ist.

Das Konfektionieren der Faser erfolgt durch gezieltes Abschneiden. Die Spitze 50 wird üblicherweise durch thermisches Ausziehen und/oder Schleifen realisiert.

Die Spitze 50 der erfindungsgemäßen Fasermikroelektrode 1 wird beispielsweise durch Anschleifen in die gewünschte Form gebracht. Durch das Schleifen werden die Elektrodenkontakte 45 so strukturiert, dass ihre Impedanz durch die größere Oberfläche von einigen 10 MOhm auf wenige MOhm (bei einer Frequenz von 1kHz) reduziert wird. Dies verringert die Dämpfung elektrischer Signale und das Elektrodenrauschen.

Zur elektrischen Kontaktierung wird der elektrische Leiter 40 an der Spitze 50, z.B. mittels Flusssäure freigelegt.

Eine bevorzugte Verwendung der erfindungsgemäßen Fasermikroelektrode liegt in der Messung von optisch herbeigeführten neuronalen Signalen. Die optische Stimulation erfolgt dabei bevorzugt durch Licht, welches durch die Fasermikroelektrode geleitet wird.

Die Anwendungsgebiete der erfindungsgemäßen Fasermikroelektrode liegen dabei insbesondere in der biomedizinischen Technik und der neurowissenschaftliche Forschung. Die erfindungsgemäße Fasermikroelektrode ermöglicht die optische Stimulation von Neuronen, was eine hochselektive Aktivierung ermöglicht. Elektrischer Strom breitet sich in biologischen Geweben kugelförmig aus. Hingegen kann Licht gebündelt und auf einen eng begrenzten Bereich fokussiert werden. Diese räumliche Selektivität kann durch genetisch geeignet transfizierte Neuronen verbessert werden. Die optische Stimulation neuronaler Strukturen verspricht weitere Innovationen, insbesondere in der biomedizinischen Technik und der Neurotechnologie, z.B. hochauflösende Implantate zur Wiederherstellung des Seh- oder Hörvermögens.

In der Biologie ist seit rund 50 Jahren bekannt, dass bestimmte Proteine (Opsine) Licht in Membranspannungsänderungen umwandeln können (Photorezeptor). Optogenetik ist die Kombination von Genetik und Optik, um Prozesse lebender Zellen zu beeinflussen. Sie umfasst die gezielte und selektive Herstellung der Lichtempfindlichkeit von Zellen (Neuronen) und von Stimulations- und Messmethoden.

Hierbei werden lichtempfindliche Proteine auf gentechnischem Wege durch Manipulation der codierenden DNA (d.h. des entsprechenden Gens) verändert und anschließend in bestimmte Zielzellen bzw. -gewebe eingebracht. Unter Lichteinfluss ist es anschließend möglich, das Verhalten der in dieser Weise modifizierten Zellen zu kontrollieren.

Es ist sogar möglich, die Lichtempfindlichkeit auf bestimmte Zonen eines Neurons zu begrenzen. Sind die Lichteigenschaften der genetisch manipulierten Zellen bekannt, dann wird eine erfindungsgemäße Fasermikroelektrode in die Nähe der optisch zu stimulierenden Zelle mittels eines Manipulators gebracht. Ein in die Faser eingekoppelter und spektro-temporal angepasster Lichtimpuls wird durch den lichtleitenden Kern zur Faserspitze übertragen. Das Licht verlässt die Faser und stimuliert die anvisierte Zelle. Diese optische Stimulation ist, im Gegensatz zur elektrischen Stimulation, hochselektiv. Die gentechnikbasierte, spezifische Herstellung lichtselektiver Zellen (Neuronen) ist nicht Gegenstand dieser Anmeldung. Sie ist dem Fachmann aber bekannt (bespielhafte Schriften zu diesem Thema: z.B. Boris V. Zemelman, Georgia A. Lee, Minna Ng und Gero Miesenböck: Selective Photostimulation of Genetically ChARGed Neurons, in: Neuron, Band 33, Nr. 1 vom 3. Januar 2002, S. 15-22. Oder Boris V. Zemelman, Nasri Nesnan, Georgia A. Lee und Gero Miesenböck: Photochemical gating of heterologous ion channels: Remote control over genetically designated populations of neurons, in: PNAS, Band 100, Nr. 3 (2003), S. 1352-1357.)

Bisherige Ableitelektroden ergeben aber nur unzureichende Ergebnisse, da sie zu weit vom Ort der Stimulation entfernt sind. Ein naheliegender, besserer und erfolgreicher Ansatz, der durch die erfindungsgemäße Fasermikroelektrode ermöglicht wird, ist es, Mikroelektroden in die Nähe der optisch zu stimulierenden neuronalen Strukturen zu bringen. Mit der erfindungsgemäßen Fasermikroelektrode kann Licht zum neuronalen Zielorgan gebracht und simultan dessen (elektrische) Antwort registriert werden.

Eine weitere Anwendungsmöglichkeit ist die optische Ableitung in Kombination mit elektrischer und/oder optischer Stimulation. Aufgrund der Ausbreitung elektrischer Ströme können mehrere Neuronen (größere Bereiche) in der Umgebung der Elektrodenkontakte stimuliert oder moduliert werden. Die Modulation kann zur Beeinflussung von neuronaler Aktivität, aber auch zur Vorbereitung effizienter Stimulation genützt werden. Dies bildet einen möglichen Ansatz zur Untersuchung und/oder Behandlung neuronaler Erkrankungen.

### Abbildungslegende und Bezugszeichenliste

**Fig. 1** Schematische Darstellung einer Ausführungsform der erfindungsgemäßen Fasermikroelektrode

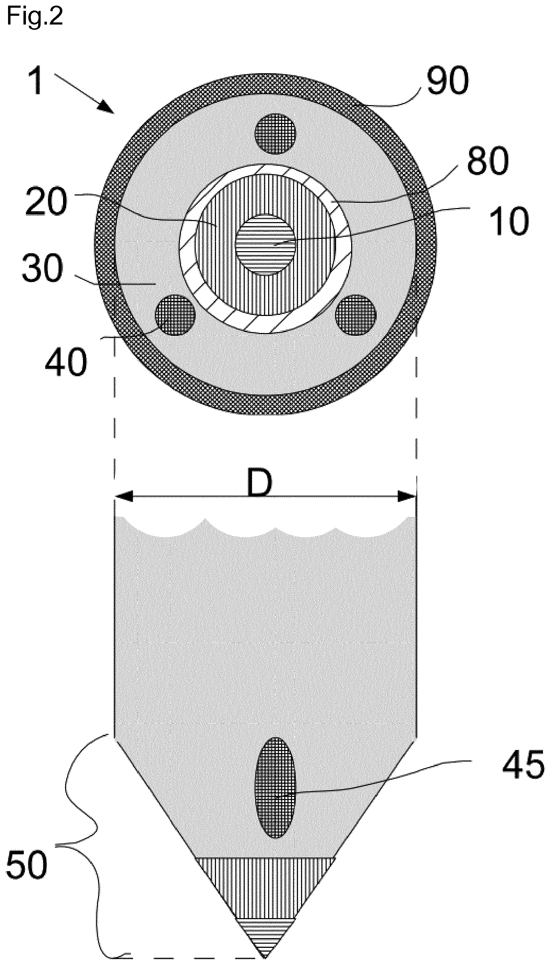

**Fig.** 2 Schematische Darstellung einer weiteren Ausführungsform der erfindungsgemäßen Fasermikroelektrode mit zusätzlicher Trennschicht 80 und Beschichtung 90.

### Bezugszeichen

1 Fasermikroelektrode
10 lichtleitender Kern
20 photonischer Kristall
30 Glasmantel
40 elektrischer Leiter
45 elektrischer Kontakt
50 Faserspitze
80 Trennschicht
90 Beschichung
D Durchmesser

## Patentansprüche

1. Fasermikroelektrode (1) umfassend wenigstens einen lichtleitenden Kern (10) und einen Glasmantel (30) um den wenigstens einen lichtleitenden Kern (10); eine Spitze (50), über die Licht in den wenigstens einen lichtleitenden Kern (10) ein- und ausgekoppelt werden kann; wenigstens einen elektrischen Leiter (40), der sich innerhalb des Glasmantels (30) befindet; wobei der wenigstens eine elektrische Leiter (40) so ausgebildet ist, dass er über die Spitze (50) elektrische Signale empfangen oder senden kann, **dadurch gekennzeichnet, dass** der wenigstens eine lichtleitende Kern (10) von einem photonischen Kristall (20) umgeben ist, wobei sich der photonische Kristall (20) zumindest abschnittsweise innerhalb des Glasmantels (30) befindet.

2. Fasermikroelektrode (1) gemäß Anspruch 1, **dadurch gekennzeichnet dass** die Fasermikroelektrode (1) eine Beschichtung (90) aufweist, welche den Glasmantel (30) zumindest abschnittsweise umhüllt.

3. Fasermikroelektrode (1) gemäß Anspruch 2, **dadurch gekennzeichnet dass** die Beschichtung (90) Acrylat, Silikon, Polyimid und/oder Parylene umfasst.

4. Fasermikroelektrode (1) gemäß Anspruch 1, **dadurch gekennzeichnet dass** die Fasermikroelektrode (1) eine Trennschicht (80) aufweist, welche den photonischen Kristall 20 zumindest abschnittsweise umhüllt.

5. Fasermikroelektrode (1) gemäß einem der vorigen Ansprüche, **dadurch gekennzeichnet dass** der photonische Kristall (20) als ein zweidimensionaler Kristall ausgebildet ist.

6. Fasermikroelektrode (1) gemäß einem der vorigen Ansprüche, **dadurch gekennzeichnet dass** der photonische Kristall (20) aus einem Halbleitermaterial, Keramik, Glas und/oder einem Polymer besteht.

7. Fasermikroelektrode (1) gemäß einem der vorigen Ansprüche, **dadurch gekennzeichnet dass** der Durchmesser D der Fasermikroelektrode (1) zwischen 15 µm bis 300 µm liegt.

## Claims

1. Fibre microelectrode (1) comprising at least one light conducting core (10) and a glass cladding (30) around the at least one light conducting core (10), a tip (50) via which light can be coupled into and out of the at least one light conducting core (10), at least one electrical conductor located inside the glass cladding (30), wherein the at least one electrical conductor (40) is designed such that it can receive or transmit electrical signals via the tip (50), **characterized in that** the at least one light conducting core (10) is surrounded by a photonic crystal (20), the photonic crystal (20) being located at least in sections within the glass cladding (30).

2. Fibre microelectrode (1) according to claim 1, **characterized in that** the fibre microelectrode (1) has a coating (90) which envelops the glass cladding (30) at least in sections.

3. Fibre microelectrode (1) according to claim 2, **characterised in that** the coating (90) comprises acrylate, silicone, polyimide and/or parylene.

4. Fibre microelectrode (1) according to claim 1, **characterised in that** the fibre microelectrode (1) has a separating layer (80) which envelops the photonic crystal (20) at least in sections.

5. Fibre microelectrode (1) according to any of the previous claims, **characterised in that** the photonic crystal (20) is formed as a two-dimensional crystal.

6. Fibre microelectrode (1) according to one of the previous claims, **characterised in that** the photonic crystal (20) consists of a semiconductor material, ceramic, glass and/or a polymer.

7. Fibre microelectrode (1) according to any of the above claims, **characterised in that** the diameter D of the fibre microelectrode (1) is between 15 µm and 300 µm.

## Revendications

1. Microélectrode en fibre (1) comprenant au moins un cœur de fibre optique (10) et une gaine de verre (30) autour d'au moins un cœur de fibre optique (10), une pointe (50) par laquelle la lumière peut être envoyée ou retenue dans au moins un cœur de fibre optique (10), au moins un conducteur électrique situé à l'intérieur de la gaine de verre (30), dans laquelle se trouve au moins un conducteur électrique (40), conçu de telle sorte qu'il peut recevoir ou transmettre des signaux électriques par la pointe (50), **caractérisé par le fait qu'**au moins un cœur de fibre optique (10) est entouré par un cristal photonique (20), le cristal photonique(20) étant situé au moins partiellement à l'intérieur de la gaine de verre (30).

2. Microélectrode en fibre (1) selon la revendication 1, **caractérisée par le fait que** la microélectrode en fibre (1) présente un revêtement (90), qui enveloppe au moins partiellement la gaine de verre (30).

3. Microélectrode en fibre (1) selon la revendication 2, **caractérisée par le fait que** le revêtement (90) comprend de l'acrylate, du silicone, du polyimide et/ou du parylène.

4. Microélectrode en fibre (1) selon la revendication 1, **caractérisée par le fait que** la microélectrode en fibre (1) présente une couche de séparation (80), qui enveloppe au moins partiellement le cristal photonique (20).

5. Microélectrode en fibre (1) selon l'une quelconque des revendications précédentes, **caractérisée par le fait que** le cristal photonique (20) est formé d'un cristal bidimentionnel.

6. Microélectrode en fibre (1) selon l'une quelconque des revendications précédentes, **caractérisée par le fait que** le cristal photonique (20) est composé d'un matériau semi-conducteur, de céramique, de verre et/ou d'un polymère.

7. Microélectrode en fibre (1) selon l'une quelconque des revendications précédentes, **caractérisée par le fait que** le diamètre D de la microélectrode en fibre (1) est compris entre 15 µm et 300 µm.
